# EUROPEAN PATENT APPLICATION

(11) **EP 4 691 252 A1**
(43) Date of publication of application: **11.02.2026**
(21) Application number: 24792835.1
(22) Date of filing: 29.02.2024
(51) Int. Cl.: A23J 1/00, A23J 1/14, A23J 1/12, A23J 3/14, A23J 3/22, C12R 1/845

(54) **METHOD FOR PRODUCING FERMENTED TEXTURIZED VEGETABLE PROTEIN WITH REDUCED OFF-FLAVOR AND OFF-ODOR**

(30) Priority: 18.04.2023 KR 20230050948
(71) Applicant: CJ Cheiljedang Corporation, Seoul 04560 (KR)
(72) Inventor: GWON, Song Hui, Seoul 04560 (KR); KANG, Ji Hyun, Seoul 04560 (KR); KIM, Mi Jung, Seoul 04560 (KR)
(74) Representative: Meissner Bolte Partnerschaft mbB
(86) International application number: PCT/KR2024/002690
(87) International publication number: WO 2024/219648

(57) **Abstract**

The present application relates to a method for producing fermented texturized vegetable protein, the method comprising a step of inoculating a substrate comprising texturized vegetable protein and starch with a fungi and culturing the same. The fermented texturized vegetable protein produced by the method of the present application has improved texture and flavor, achieving a meat-like mouthfeel, and thus can be used as an alternative meat material.

## Description

### TECHNICAL FIELD

### [Cross-Reference with Related Applications]

The present application claims the benefit of priority from Korean Patent Application No. 10-2023-0050948, filed on April 18, 2023, the entire contents of which are incorporated herein as part of this specification.

### [TECHNICAL FIELD]

The present application relates to a fermented texturized vegetable protein with reduced off-flavor and off-odor, a method for producing the same, a meat alternative comprising the same, and a method for reducing the off-flavor or off-odor of the fermented texturized vegetable protein.

### BACKGROUND ART

Plant-based meat is a food produced to taste like meat using plant-based ingredients rather than animal-based ingredients such as meat, and is also called plant-based meat alternative. Plant-based meat includes wheat meat made using gluten, a protein contained in wheat flour, rice meat made using rice, and soy meat made by mixing soy protein and gluten.

In order to make soy protein have a meat texture, a spinning process, thermoplastic extrusion, steam texturization, etc. have been used. In thermoplastic extrusion and steam texturization, a true fibrous structure is not formed, but soy proteins are hydrated and form layers to produce a chewy material.

A texturized vegetable protein (TVP) is produced through thermoplastic extrusion using soy protein as the main ingredient. When defatted soy flour or concentrated soy protein is mixed well with water and then heated in an extruder and extruded under high pressure, the soy protein molecules coagulate with directionality, giving the tissue a chewing sensation similar to meat. The texture and taste of the final texturized vegetable protein product are significantly affected by the type and ratio of mixed ingredients, moisture content of mixed ingredients, heating temperature, and heating time. Texturized vegetable protein (TVP), which is commercially available at present, is a low-moisture TVP in the form of a pellet. It is produced into a lump form by adding a binder after hydration and is used in food.

When a texturized vegetable protein (TVP) is used as a raw material for plant-based meat, it must have the same texture, flavor, and appearance as meat, and the texture must not be destroyed by rehydration, which is essential during cooking, and it must also possess functionalities such as water-retention capacity and fat absorption comparable to those of meat. However, since TVP has a lower texture than meat and has unique off-flavor and off-odor derived from plant raw materials, it has insufficient characteristics to be used as a raw material for meat alternatives. Korean Patent No. 10-2460066 describes a method for producing artificial meat with a meat-like texture by reacting a mixture of vegetable protein, gluten, starch, a protein cross-linking agent, and protein hydrolysate, mixing with an oil, and discharging the resultant through a micro-nozzle so that the layered structures bind to one another. However, this document does not disclose a technique for improving the binding strength by inoculating and fermenting fungi into a texturized vegetable protein (TVP) without using a binder, while reducing the off-flavor and off-odor generated by the fermentation of fungi.

### [Prior Art Documents]

### [Patent Document]

Korean Patent No. 10-2460066

### DISCLOSURE OF THE INVENTION

### TECHNICAL PROBLEM

In order to utilize a texturized vegetable protein (TVP) as a meat alternative material, the present inventors have made research efforts to achieve the formation of a binding in the TVP through fermentation without the addition of any necessary binder thereby allowing a meat alternative to have a texture similar to a meat-containing product, while simultaneously reducing the off-odor that may be generated by the fermentation. As a result, the present inventors have experimentally demonstrated that the above-mentioned object can be achieved when a TVP culture is produced by inoculating fungi on a mixed substrate, which is prepared by adding starch to a texturized vegetable protein (TVP), and culturing the same, thereby completing the present application.

Accordingly, an object of the present application is to provide a fermented texturized vegetable protein (TVP) with an improved texture and reduced off-flavor and off-odor, a method for producing the same, a meat alternative comprising the same, a method for reducing the off-flavor or off-odor of a fermented texturized vegetable protein, and a use of a substrate comprising a texturized vegetable protein (TVP) and starch for the reduction of the off-flavor or off-odor of a fermented texturized vegetable protein.

### TECHNICAL SOLUTION

An aspect of the present application provides a method of producing a fermented texturized vegetable protein, comprising inoculating and culturing fungi on a substrate comprising a texturized vegetable protein (TVP) and starch.

In the present application, fungi are inoculated and cultured on a substrate comprising a texturized vegetable protein (TVP) and starch.

The term "texturized vegetable protein (TVP)" used in the specification of the present application refers to a protein raw material whose main component is a protein derived from a plant.

The raw material of the texturized vegetable protein of the present application may comprise a protein derived from wheat, soybeans, peas, sesame seeds, cotton seeds, or rice, and specifically may comprise a protein derived from soybeans, wheat, or peas. Specifically, the texturized vegetable protein in the present specification may refer to a texturized vegetable protein produced using a soybean protein as a main raw material through an extrusion molding process. The texturized vegetable protein (TVP) may be produced by mixing defatted soy flour or a concentrated soy protein well with water and then extruding the mixture at a high pressure while heating in an extruder. The texturized vegetable protein (TVP) of the present application may have the same meaning as a texturized soy protein (TSP), soybean meat, and a soy protein in a broad sense.

The texturized vegetable protein may have various sizes and shapes depending on the shape of the extruder's injection port and the temperature and pressure conditions during extrusion. For example, the texturized vegetable protein may be in the form of chunks, flakes, granula, minced, slices, or strips, and the size of the texturized vegetable protein may be 5 mm to 60 mm in length and 1 mm to 20 mm in thickness, but is not limited thereto.

In the present application, starch included in the substrate refers to a polymer carbohydrate in which glucose units are linked by glycosidic bonds.

The starch may comprise starch present in raw plants extracted in powder form.

In an embodiment, the plant from which the starch is extracted is not particularly limited as long as it is a plant in which starch exists, and for example, starch extracted from any one raw material plant selected from the group consisting of wheat, rice, glutinous rice, mung beans, barley, soybeans, corn, sorghum, potato, sweet potato, tapioca, cassava, and arrowroot may be used. Specifically, rhizome starch may be used, and more specifically, corn starch, potato starch, and tapioca starch may be used.

The starch comprises starch including normal starch (unprocessed starch), pregelatinized starch, or modified starch.

In the present application, normal starch refers to starch that maintains the inherent properties of starch, and is contrast to processed starch.

In the present application, pregelatinized starch refers to starch obtained by pregelatinization treatment of normal starch. Pregelatinization of starch refers to the phenomenon where, when water and heat are applied to starch, a starch dispersion forms a transparent or milky white colloidal solution with very high viscosity as the temperature rises. When the concentration is high or the solution is cooled, it forms a semi-solid gel. This phenomenon is also referred to as pregelatinization, colloidization, or gelatinization of starch. That is, beta starch having a regular molecular arrangement changes into pregelatinized starch having an irregular molecular arrangement. The starch to be pregelatinized may comprise the above-mentioned normal starch, and specifically, may be starch extracted from one selected from the group consisting of wheat, rice, glutinous rice, corn, potato, sweet potato, tapioca, cassava, or arrowroot. Specifically, it may be pregelatinized corn starch or waxy corn gelatinized starch.

In the present application, modified starch refers to starch that has been chemically modified. In an embodiment of the present application, the modified starch may comprise all starches that have been chemically modified from various grains or rhizomes. Specifically, the modified starch may be a chemically modified version of the above-described normal starch, and more specifically, may include oxidized starch, acetyl starch, ester starch, ether starch, cross-linked starch, cross-linked ester starch, or cross-linked ether starch of one or more starches selected from the group consisting of tapioca starch, corn starch, waxy corn starch, potato starch, non-glutinous rice starch, and glutinous rice starch. Specifically, the modified starch may include modified starch of tapioca starch, and more specifically, the modified starch may include one or more among hydroxypropyl distarch phosphate, distarch phosphate, starch acetate, oxidized starch, and acetylated adipate starch of tapioca starch.

As described above, the starch may be obtained by extraction and purification from a raw material plant in which starch exists, but it also includes the raw material plant itself in which starch exists without an extraction or purification process. The raw material plant itself in which starch exists may be, for example, a powder of a raw material plant containing starch, and specifically, may be a plant powder having a starch content of 40 wt% or more, 50 wt% or more, 60 wt% or more, 70 wt% or more, or 80 wt% or more relative to the total powder weight on a dry weight basis, and more specifically, the starch in the present application may include, but is not limited to, corn powder, potato powder, sweet potato powder, tapioca powder, oat powder, rice powder, acorn powder, barley powder, or rye powder having the above-mentioned starch content relative to the total powder weight on a dry weight basis.

In the present application, the weight ratio of texturized vegetable protein (TVP) and starch may be TVP: starch = 55: 45 to 90: 10, and specifically, the weight ratio may be TVP: starch = 56: 44 to 89: 11, 57: 43 to 88: 12, 57: 43 to 87: 13, 59: 41 to 87: 13, 62: 38 to 85: 15, 65: 35 to 80: 20, 68: 32 to 80: 20, 70: 30 to 80: 20, 73: 26 to 80: 20, 73: 26 to 85: 15, 73: 26 to 87: 13, but is not limited thereto.

In the present application, when the weight ratio of a texturized vegetable protein and starch is within the above-identified range, the content of ammonium ions in the produced fermented texturized vegetable protein is reduced compared to the content of ammonium ions in the fermented texturized vegetable protein produced without adding starch to the substrate, and simultaneously there is an effect in that the texture of the fermented texturized vegetable protein is improved. The lower the content of ammonium ions, which are the cause of off-flavor and off-odor in the fermented texturized vegetable protein, the more significantly the off-flavor and off-odor are reduced.

In addition, when the starch is added in an excessively large amount such that the weight ratio of starch to TVP in the substrate of the present application exceeds 45, there is a disadvantage in that the shape of TVP grains or granules before fermentation is not well maintained, and as a result, fungal mycelia are not uniformly formed in the fermented texturized vegetable protein (TVP).

In the present application, in a substrate comprising a texturized vegetable protein (TVP) and starch, the starch may be included in an amount of 5-50 wt%, 5-45 wt%, 5-40 wt%, 5-35 wt%, 5-30 wt%, 7-50 wt%, 7-45 wt%, 7-40 wt%, 7-35 wt%, 7 -30 wt%, 10-50 wt%, 10-45 wt%, 10-40 wt%, 10-35 wt%, 10-30 wt%, or 12-27 wt% relative to the total substrate, but is not limited thereto.

In an embodiment, a monosaccharide or disaccharide may not be added to a substrate comprising a texturized vegetable protein and starch.

The monosaccharide not added to the substrate may be glucose, fructose, or galactose, and the disaccharide not added to the substrate may be sucrose, lactose, or maltose.

Not adding monosaccharide or disaccharide to the substrate refers to not additionally or artificially adding the above-mentioned sugars to the substrate; therefore, it does not exclude the inclusion of a certain amount of sugar that is inherently contained in a substance included in the substrate, such as a texturized vegetable protein, starch, and a fungal culture inoculated onto the substrate. Specifically, the substrate may include a small amount of a monosaccharide or disaccharide inherently contained in the substance included in the substrate, and the content of such monosaccharide and disaccharide may be, for example, less than 7 wt%, less than 6 wt%, less than 5 wt%, less than 4 wt%, less than 3 wt%, less than 2 wt%, less than 1 wt%, less than 0.7 wt%, less than 0.5 wt%, less than 0.3 wt%, less than 0.2 wt%, or less than 0.1 wt% relative to the total substrate.

In the present application, the fungi used for inoculation onto a substrate and cultivation may comprise edible fungi capable of forming mycelia.

In an embodiment, the fungi may be at least one species selected from the group consisting of fungi of the genus *Rhizopus,* fungi of the genus *Mucor,* fungi of the genus *Neurospora,* fungi of the genus *Amylomyces,* fungi of the genus *Aspergillus,* and fungi of the genus *Monascus.*

In another embodiment, the *Rhizopus* fungi may be *Rhizopus oligosporus.*

In an embodiment, the fungi may be used by direct inoculation into a substrate comprising a texturized vegetable protein and starch.

In an embodiment, the method of the present application may further comprise a step of adjusting the moisture content by adding water to the substrate comprising a texturized vegetable protein and starch before inoculating the substrate with the fungi. The moisture content may be 55-75 wt%, 57-73 wt%, or 60-70 wt% based on 100 wt% of the total substrate. Additionally, the substrate to which water has been added may be kept at room temperature to hydrate the substrate.

In an embodiment, the method of the present application may further comprise a step of sterilizing the substrate after the step of adjusting the moisture content by adding water to the substrate.

In an embodiment, the sterilization may be performed by heating at a temperature of 110°C to 125°C for 5-15 minutes.

In an embodiment, the amount of fungi inoculated onto the substrate may be 0.5 × 10⁴ cfu/g to 0.5 x 10⁸ cfu/g, or 0.5 × 10⁵ cfu/g to 0.5 × 10⁷ cfu/g.

In the present application, the conditions for culturing after inoculating fungi on a substrate may be selected appropriately depending on the type of fungi. For example, after inoculating fungi on a substrate, the inoculated substrate may be cultured under temperature conditions of 25-35°C and humidity conditions of 40% to 99% for a time ranging from 1 hour to 10 days.

In the present application, when fungi are inoculated and cultured on a substrate comprising a texturized vegetable protein and starch, the fungal mycelia grow and attach between the pores of the texturized vegetable protein. This growth forms the binding strength of the texturized vegetable protein (TVP). Furthermore, the addition of starch allows the fungi to be cultured, resulting in the effect of reducing the off-flavor and off-odor of the fermented texturized vegetable protein.

The fermented texturized vegetable protein produced by the present method may exhibit a reduced off-flavor or off-odor, and the reduction in off-flavor or off-odor may be expressed by the content of ammonium ions in the fermented texturized vegetable protein, and specifically, the content of ammonium ions in the fermented texturized vegetable protein may be characterized as a low content of 5000 µg/g or less.

Specifically, the produced fermented texturized vegetable protein may comprise ammonium ions in a content of 5000 µg/g or less, 4500 µg/g or less, 4000 µg/g or less, 3500 µg/g or less, 3300 µg/g or less, 3200 µg/g or less, 3100 µg/g or less, 3000 µg/g or less, 2700 µg/g or less, 2500 µg/g or less, 2300 µg/g or less, 2000 µg/g or less, 1900 µg/g or less, 1800 µg/g or less, 1700 µg/g or less, 1500 µg/g or less, 1300 µg/g or less, 1100 µg/g or less, 1000 µg/g or less, or 900 µg/g or less.

Specifically, the produced fermented texturized vegetable protein may comprise ammonium ions in a content of 500-3,500 µg/g, and specifically, it may comprise ammonium ions in a content of 500-3,500 µg/g, 600-3,500 µg/g, 700-3,500 µg/g, 800-3,500 µg/g, 900-3,500 µg/g, 500-3,300 µg/g, 600-3,300 µg/g, 700-3,300 µg/g, 800-3,300 µg/g, 900-3,300 µg/g, 500-3,100 µg/g, 600-3,100 µg/g, 700-3,100 µg/g, 800-3100 µg/g, 900-3100 µg/g, 910-3100 µg/g, 920-3100 µg/g, 930-3100 µg/g, 940-3100 µg/g, 950-3100 µg/g, 910-3000 µg/g, 920-3000 µg/g, 930-3000 µg/g, 940-3000 µg/g, or 950-3000 µg/g.

The fermented texturized vegetable protein produced by the present method may have excellent textural characteristics. This characteristics may comprise one or more of the following textural characteristics: (i) Hardness of 400-3000 (gf), (ii) Elasticity of 0.4 to 0.8, (iii) Cohesiveness of 0.2 to 0.44, and (iv) Chewiness of 121 to 900. The contents of the textural characteristics are the same as described below.

Another aspect of the present application provides a fermented texturized vegetable protein obtained by inoculating fungi into a substrate comprising a texturized vegetable protein (TVP) and starch and culturing the same.

In an embodiment, the fermented texturized vegetable protein may comprise ammonium ions in a content of 5000 µg/g or less, and specifically, the fermented texturized vegetable protein may comprise ammonium ions in a content of 5000 µg/g or less, 4500 µg/g or less, 4000 µg/g or less, 3500 µg/g or less, 3300 µg/g or less, 3200 µg/g or less, 3100 µg/g or less, 3000 µg/g or less, 2700 µg/g or less, 2500 µg/g or less, 2300 µg/g or less, 2000 µg/g or less, 1900 µg/g or less, 1800 µg/g or less, 1700 µg/g or less, 1500 µg/g or less, 1300 µg/g or less, 1100 µg/g or less, 1000 µg/g or less, or 900 µg/g or less.

Specifically, the fermented texturized vegetable protein may comprise ammonium ions in a content of 500-3,500 µg/g, and specifically, may comprise ammonium ions in a content of 500-3,500 µg/g, 600-3,500 µg/g, 700-3,500 µg/g, 800-3,500 µg/g, 900-3,500 µg/g, 500-3,300 µg/g, 600-3,300 µg/g, 700-3,300 µg/g, 800-3,300 µg/g, 900-3,300 µg/g, 500-3,100 µg/g, 600-3,100 µg/g, 700-3,100 µg/g, 800-3100 µg/g, 900-3100 µg/g, 910-3100 µg/g, 920-3100 µg/g, 930-3100 µg/g, 940-3100 µg/g, 950-3100 µg/g, 910-3000 µg/g, 920-3000 µg/g, 930-3000 µg/g, 940-3000 µg/g, or 950-3000 µg/g.

In an embodiment, the fermented texturized vegetable protein of the present application may have one or more of the following textural characteristics: (i) Hardness of 400-3000 (gf), (ii) Elasticity of 0.4 to 0.8 (ratio-T), (iii) Cohesiveness of 0.2 to 0.44 (ratio-A), and (iv) Chewiness of 121 to 900.

More specifically, the fermented texturized vegetable protein of the present application may have textural properties of any one or a combination of the following:
(i) a hardness of 400-3000 (gf), specifically, a hardness of 400-3000 (gf), 400-2900 (gf), 400-2850 (gf), 400-2800 (gf), 400-2700 (gf), 400-2600 (gf), 400-2500 (gf), 400-2300 (gf), 400-2100 (gf), 400-1900 (gf), 400-1700 (gf), 400-1500 (gf), or 400-1400 (gf);
(ii) an elasticity of 0.4 to 0.8, specifically, an elasticity of 0.4 to 0.8, 0.4 to 0.79, 0.4 to 0.78, 0.4 to 0.77, 0.4 to 0.76, 0.4 to 0.74, 0.43 to 0.8, 0.43 to 0.79, 0.43 to 0.78, 0.43 to 0.77, 0.43 to 0.76, 0.43 to 0.74, 0.45 to 0.8, 0.45 to 0.79, 0.45 to 0.78, 0.45 to 0.77, 0.45 to 0.76, 0.45 to 0.74, 0.47 to 0.8, 0.47 to 0.79, 0.47 to 0.78, 0.47 to 0.77, 0.47 to 0.76, 0.47 to 0.74, 0.5 to 0.8, 0.5 to 0.79, 0.5 to 0.78, 0.5 to 0.77, 0.5 to 0.76, 0.5 to 0.74, 0.52 to 0.8, 0.52 to 0.79, 0.52 to 0.78, 0.52 to 0.77, 0.52 to 0.76, or 0.52 to 0.74;
(iii) a cohesiveness of 0.2 to 0.44, specifically, a cohesiveness of 0.2 to 0.44, 0.22 to 0.44, 0.24 to 0.44, 0.26 to 0.44, 0.28 to 0.44, 0.3 to 0.44, 0.32 to 0.44, or 0.33 to 0.44; and
(iv) a chewiness of 121 to 900, specifically, a chewiness of 121 to 900, 121 to 850, 121 to 800, 121 to 750, 121 to 700, 121 to 650, 121 to 600, 121 to 550, 121 to 500, 121 to 450, or 121 to 410.

The above-identified numerical values of hardness, elasticity, cohesiveness, and chewiness are the numerical values measured when the properties of a fermented texturized vegetable protein sample are measured using a texture analyzer under the following conditions:
Probe: A cylindrical probe with a diameter of 2 cm;
Speed at which the probe descends to the sample (Pre-Test Speed): 2 mm/sec;
Speed at which the probe penetrates the sample after touching the sample surface (Test Speed): 2 mm/sec;
The speed at which the probe returns to its original position after penetrating the sample (Post-Test Speed): 2 mm/sec;
Target Mode of the probe: Strain;
Strain: 55%;
Conditions for the above probe to recognize the sample (Trigger Type Auto): Force; and
Conditions set to the minimum force (Trigger Force) for the probe to recognize the presence of the sample: 5 g.

The hardness refers to the force required to reach deformation and may be measured as the highest force at the first compression.

The elasticity refers to the property of a deformed sample to return to its original state after the force is removed, and may be measured as the height of the sample to which it recovers after the first compression.

The cohesiveness refers to the strength of the sample to maintain its original shape, and may be calculated as a ratio calculated by dividing the area during the second compression by the area during the first compression.

The chewiness refers to the property of making a solid sample swallowable, and may be calculated as "hardness × elasticity × cohesiveness".

In an embodiment, the fermented texturized vegetable protein produced in the present application may simultaneously have all of one or more of the above-described textural characteristics selected from (i) the content of the ammonium ions described above; and (ii) the hardness, elasticity, cohesiveness, and chewiness described above.

The raw material of the texturized vegetable protein of the present application may comprise a protein derived from wheat, soybeans, peas, sesame seeds, cotton seeds, or rice, and specifically, may comprise a protein derived from soybeans, wheat, or peas. Specifically, the texturized vegetable protein may refer to a texturized vegetable protein produced through an extrusion molding process using a soybean protein as a main raw material. The texturized vegetable protein (TVP) may be produced by mixing defatted soy flour or a concentrated soy protein well with water and then extruding the mixture at high pressure while heating in an extruder. The texturized vegetable protein (TVP) of the present application may have the same meaning as a texturized soy protein (TSP), soybean meat, and a soy protein in a broad sense. The texturized vegetable protein may have various sizes and shapes depending on the shape of the extruder's injection port and the temperature and pressure conditions during extrusion. For example, the form of the texturized vegetable protein may be chunks, flakes, granules, minced, slices, or strips, and the size of the texturized vegetable protein may be, for example, a length of 5-60 mm and a thickness of 1-20 mm, but is not limited thereto.

In the present application, the starch contained in the substrate refers to a polymer carbohydrate in which glucose units are linked by glycosidic bonds.

The starch may be starch present in raw plants extracted in powder form.

In an embodiment, the plant from which the starch is extracted is not particularly limited as long as it is a plant in which starch exists. For example, the starch extracted from one raw material plant selected from the group consisting of wheat, rice, glutinous rice, mung beans, barley, soybeans, corn, sorghum, potato, sweet potato, tapioca, cassava, and arrowroot may be used. Specifically, rhizome starch may be used, and more specifically, corn starch, potato starch, and tapioca starch may be used.

The starch comprises starch which contains normal starch, pregelatinized starch, or modified starch.

In the present application, normal starch refers to starch that maintains the inherent properties of starch, and is contrast to processed starch.

The pregelatinized starch refers to starch obtained by pregelatinization treatment of normal starch, and the starch to be pregelatinized may be the normal starch described above. Specifically the normal starch may comprise starch extracted from one selected from the group consisting of wheat, rice, glutinous rice, corn, potato, sweet potato, tapioca, cassava, and arrowroot. Specifically, it may be pregelatinized corn starch or waxy corn gelatinized starch.

The modified starch refers to starch that has been chemically modified, and specifically may comprise oxidized starch, acetyl starch, ester starch, ether starch, cross-linked starch, cross-linked ester starch, or cross-linked ether starch of one or more starches selected from the group consisting of tapioca starch, corn starch, waxy corn starch, potato starch, non-glutinous rice starch, and glutinous rice starch.

As described above, the starch may be obtained by extraction and purification from a raw material plant in which starch exists, but it also includes the raw material plant itself in which starch exists without an extraction or purification process. The raw material plant itself in which starch exists may be, for example, a powder of a raw material plant containing starch, and specifically, may be a plant powder having a starch content of 40 wt% or more, 50 wt% or more, 60 wt% or more, 70 wt% or more, or 80 wt% or more relative to the total powder weight on a dry weight basis, and more specifically, may comprise corn powder, potato powder, sweet potato powder, tapioca powder, oat powder, rice powder, acorn powder, barley powder, or rye powder having the above-mentioned starch content relative to the total powder weight on a dry weight basis, but is not limited thereto.

The weight ratio of the texturized vegetable protein (TVP) and starch may be TVP: starch = 55:45 to 90:10, and specifically, the weight ratio may be TVP: starch = 56:44 to 89:11, 57:43 to 88:12, 57:43 to 87:13, 59:41 to 87:13, 62:38 to 85:15, 65:35 to 80:20, 68:32 to 80:20, 70:30 to 80:20, 73:26 to 80:20, 73:26 to 85:15, 73:26 to 87:13, but is not limited thereto.

In the substrate comprising TVP and starch, the starch may be included in an amount of 5-50 wt%, 5-45 wt%, 5-40 wt%, 5-35 wt%, 5-30 wt%, 7-50 wt%, 7-45 wt%, 7-40 wt%, 7-35 wt%, 7-30 wt%, 10-50 wt%, 10-45 wt%, 10-40 wt%, 10-35 wt%, 10-30 wt%, or 12-27 wt% relative to the total substrate, but is not limited thereto.

In an embodiment, the substrate comprising texturized vegetable protein(TVP) and starch may not comprise a monosaccharide or disaccharide added thereto.

In another embodiment, the fermented TVP of the present application may not comprise a monosaccharide or disaccharide added thereto.

The monosaccharide not added to the substrate or fermented TVP may be glucose, fructose, or galactose, and the disaccharide not added to the substrate or fermented TVP may be sucrose, lactose, or maltose.

Not adding a monosaccharide or disaccharide to the substrate or fermented TVP refers to not additionally or artificially adding the above-mentioned sugars to the substrate; therefore, it does not exclude the inclusion of a certain amount of sugar inherently contained in a substance included in the substrate or fermented TVP, such as texturized vegetable protein (TVP), starch, and a fungal culture inoculated onto the substrate, a fungus inoculated onto the substrate and cultured, and by-products. Specifically, the substrate or fermented TVP may comprise a small amount of monosaccharides or disaccharides inherently contained in the substances included therein, and the content of such monosaccharides and disaccharides may be, for example, less than 7 wt%, less than 6 wt%, less than 5 wt%, less than 4 wt%, less than 3 wt%, less than 2 wt%, less than 1 wt%, less than 0.7 wt%, less than 0.5 wt%, less than 0.3 wt%, less than 0.2 wt%, or less than 0.1 wt% relative to the total substrate or fermented texturized vegetable protein.

In the present application, the fungi used for culturing by inoculating the substrate may comprise an edible fungi capable of forming mycelia. In an embodiment, the fungi may be at least one selected from the group consisting of fungi of the genus *Rhizopus,* fungi of the genus *Mucor,* fungi of the genus *Neurospora,* fungi of the genus *Amylomyces,* fungi of the genus *Aspergillus,* and fungi of the genus *Monascus.* In another embodiment, the fungi of the genus *Rhizopus* may be *Rhizopus oligosporus.*

In an embodiment, the fermented TVP of the present application includes mycelia produced during culturing of the fungi.

Another aspect of the present application provides a method for reducing off-flavor or off-odor of fermented TVP (TVP), comprising the step of inoculating and culturing fungi on a substrate comprising TVP and starch.

In an embodiment, the TVP may comprise a protein derived from wheat, soybeans, peas, sesame seeds, cotton seeds, or rice.

In an embodiment, the starch comprises normal starch, pregelatinized starch, or modified starch.

In an embodiment, the normal starch may comprise starch extracted from one selected from the group consisting of wheat, rice, glutinous rice, mung beans, barley, soybeans, corn, sorghum, potato, sweet potato, tapioca, cassava, and arrowroot.

In an embodiment, the weight ratio of the texturized vegetable protein (TVP) and starch may be TVP:starch = 55:45 to 90:10. Specifically, in the present application, the weight ratio of TVP and starch may be TVP:starch = 55:45 to 90:10, and specifically, the weight ratio of TVP:starch may be 55 : 45 to 90 : 10, 56 : 44 to 89 : 11, 57 : 43 to 88 : 12, 57 : 43 to 87 : 13, 59 : 41 to 87 : 13, 62 : 38 to 85 : 15, 65 : 35 to 80 : 20, 68 : 32 to 80 : 20, 70 : 30 to 80 : 20, 73 : 26 to 80 : 20, 73 : 26 to 85 : 15, 73 : 26 to 87 : 13, but is not limited to thereto.

In another aspect of the present application providing a method for reducing the off-flavor or off-odor of fermented TVP, each step and each component are the same as those described in the above other aspects of the present application, fermented TVP and a preparation method thereof; therefore, the detailed description thereof is substituted by the preceding description.

In still another aspect of the present application, there is provided a meat alternative comprising the fermented TVP described above.

The fermented TVP of the present application may be utilized as a meat alternative material due to its improved texture and flavor. Foods that may be utilized as a meat alternative material include, but are not limited to, patties, meatballs, nuggets, tenders, fish cutlets, pork cutlets, or sweet and sour pork.

In still another aspect of the present application, there is provided a use of a substrate comprising a texturized vegetable protein (TVP) and starch for reducing the off-flavor or off-odor of a fermented texturized vegetable protein, wherein the fermented TVP provides a use in which the fermented texturized vegetable protein is produced by inoculating and culturing fungi on the substrate.

In an embodiment, the TVP may comprise a protein derived from wheat, soybeans, peas, sesame seeds, cotton seeds, or rice.

In an embodiment, the starch may comprise normal starch, pregelatinized starch, or modified starch.

In an embodiment, the normal starch may be starch extracted from one selected from the group consisting of wheat, rice, glutinous rice, mung beans, barley, soybeans, corn, sorghum, potato, sweet potato, tapioca, cassava, and arrowroot.

In an embodiment, the weight ratio of the texturized vegetable protein (TVP) and starch may be TVP:starch = 55:45 to 90:10.

In the use of the substrate comprising a texturized vegetable protein (TVP) and starch for reducing the off-flavor or off-odor of a fermented texturized vegetable protein, which is another aspect of the present application, each step and each component are the same as those described in the above other aspects of the present application, fermented TVP and a preparation method thereof; therefore, the detailed description thereof is substituted by the preceding description.

### ADVANTAGEOUS EFFECTS

According to the method of the present application, by inoculating fungi on a substrate comprising a texturized vegetable protein (TVP) and starch and culturing the same, the texture of the fermented TVP produced is improved, and simultaneously, the content of ammonium ions is lowered, thereby reducing the off-flavor and off-odor produced by fermentation or derived from plant raw materials. The fermented TVP produced by the method of the present application exhibits improved texture and flavor, providing a meat-like texture, and can thus be used as a meat substitute.

However, the effects of the present application are not limited to the effects mentioned above, and other effects not mentioned will be clearly understood by those skilled in the art from the description below.

### BRIEF DESCRIPTION OF THE DRAWINGS

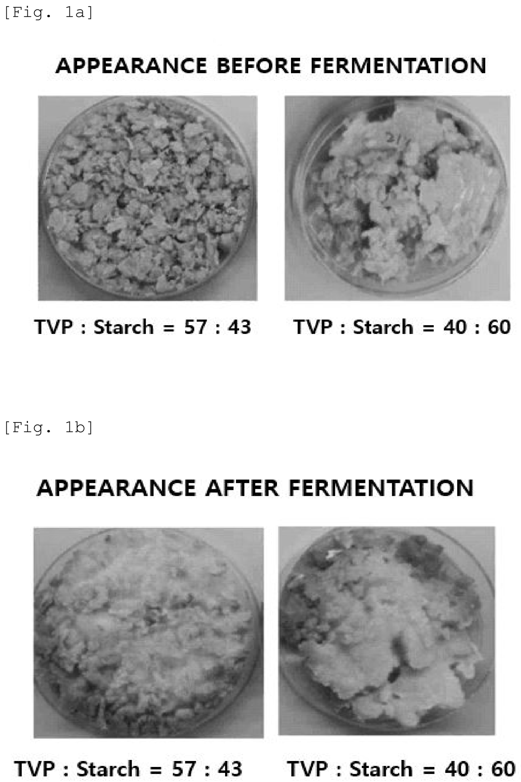

FIG. 1a shows images of the appearance of a substrate before fermentation, the substrate comprising a texturized vegetable protein (TVP) and starch in a weight ratio of 57:43 or 40:60, respectively.
FIG. 1b shows images of the appearance of fermented texturized vegetable protein (TVP) by inoculating the fungi *Rhizopus oligosporus* into a substrate comprising TVP and starch in a weight ratio of 57:43 or 40:60, respectively.

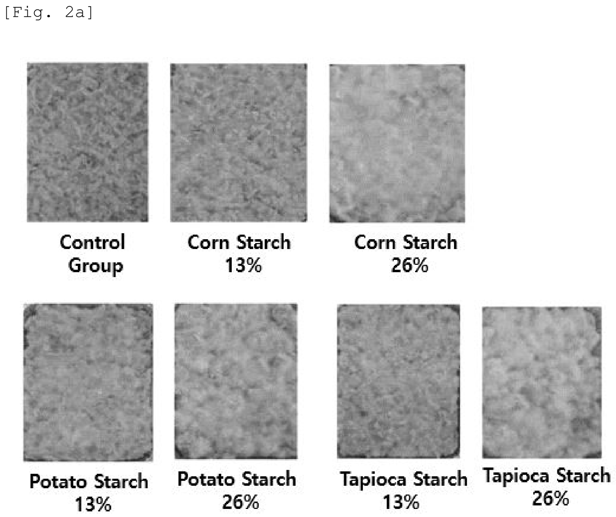

FIG. 2a shows images of the appearance of fermented texturized vegetable protein (TVP) by inoculating the fungi *Rhizopus oligosporus* onto a TVP substrate to which starch was not added (control), a substrate to which 13 wt% and 26 wt% of corn starch was added to TVP, a substrate to which 13 wt% and 26 wt% of potato starch was added to TVP, and a substrate to which 13 wt% and 26 wt% of tapioca starch was added to TVP.

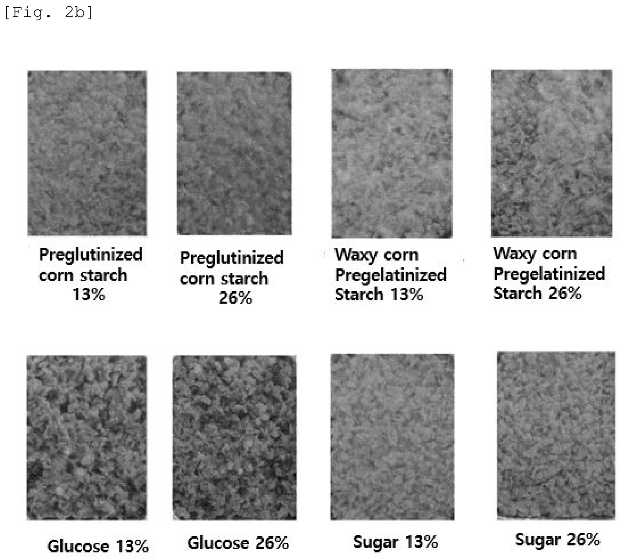

FIG. 2b shows images of the appearance of fermented TVP, which was fermented by inoculating the fungi *Rhizopus oligosporus* onto a substrate to which 13 wt% and 26 wt% of pregelatinized corn starch added to TVP, a substrate to which 13 wt% and 26 wt% of waxy corn gelatinized starch was added to TVP, a substrate to which 13 wt% and 26 wt% of glucose was added to TVP, and a substrate to which 13 wt% and 26 wt% of sugar was added to TVP, respectively.

### MODE FOR CARRYING OUT THE INVENTION

Hereinafter, the present application will be described in detail by way of examples. However, the following examples are intended to specifically illustrate the present application, and the content of the present application is not limited by the following examples.

### [Example]

### [Example 1] Preparation of fermented texturized vegetable protein (TVP)

### 1. Isolation of strains

A *Rhizopus oligosporus* strain was isolated from soybean paste (meju), cultured on Potato Dextrose Agar (PDA) medium at 30°C for 4 days, and then preserved at 4°C. The preserved strain was used in the experiment.

### 2. Preparation of inoculation source

The inoculum was used by isolating a portion of the strain that had been preserved and inoculating it into sterilized tofu bean curd and cultivating it at 30°C for 4 days. The bean curd used for inoculation was set to have a moisture content of 60% and was sterilized at 121°C for 15 minutes. The bean curd on which the strain had been cultured was dried and then pulverized in a pulverizer (Comac, ME2) before use.

### 3. Main culture

A texturized vegetable protein (TVP) was purchased as Contex 31 TVP from Solbar (Solbar Ningbo Protein Technology Co., Ltd.). Contex 31 is a flake-type TVP with a diameter of about 5-15 mm using soy protein concentrate as its active ingredient.

Corn starch (Farmer's Garden, content 99.5%), potato starch (Chaiwell, content 100%), tapioca starch (Biomix, 99.9%), pregelatinized corn starch (Daesang, content corn starch 100%), waxy corn pregelatinized starch (Rocket), glucose (Hwa-mi, glucose), and white sugar (CJ CheilJedang, white sugar) were purchased from each vendor.

To the above-mentioned prepared TVP, one of the starches described above, glucose, or white sugar was added, mixed and homogenized, and used as a substrate. When mixing, the mixing ratio of TVP and carbohydrate was adjusted to a weight ratio of TVP: carbohydrate = 87:13 or 73:26. Water was added to the homogenized substrate so that the moisture content became 65 wt%, and mixed. The substrate with the added moisture was hydrated by leaving it at room temperature for about 30 minutes. The hydrated substrate was sterilized at 121°C for 15 minutes to block possible contamination sources derived from the substrate.

After cooling the prepared substrate to below 30°C, the prepared inoculum was finally inoculated with *Rhizopus oligosporus.* The substrate was inoculated so that the bacterial count was 5 × 10⁵ cfu/g.

After inoculating the substrate with the inoculum, the substrate was homogenized, placed in a tray with ventilation holes, sealed, and cultured under humid conditions of 30°C for 17 hours. The cultured TVP was produced in the form of a lump in which the TVP particles were bound together by evenly growing mycelia of the fungus between the TVP particles. The final TVP fungal culture was stored in a refrigerator to stop the fermentation process.

### [Example 2] Texture analysis of fermented TVP

The texture of the TVP fungal culture was measured using a texture analyzer (TA-XT plus, Stable Micro Systems, England). Texture profile analysis (TPA) was performed under the conditions described in Table 1 below. The size of the TVP fungal culture sample was set to 1.3 mm × 1.3 mm × 1.3 mm (width × length × height), and the compression ratio was set to 55%. Hardness represents the highest force at the first compression, elasticity is the height of the sample recovered after the first compression, cohesiveness is the ratio calculated by dividing the area at the second compression by the first compression, and chewiness is expressed as "hardness × elasticity × cohesiveness".

**[Table 1]**

| Division | Analysis Conditions |
|---|---|
| Pre-Test Speed | 2 mm/sec |
| Test Speed | 2 mm/sec |
| Post-Test Speed | 2 mm/sec |
| Target Mode | Strain |
| Strain | 55% |
| Trigger Type | Auto (Force) |
| Trigger Force | 5 g |

### [Example 3] Ammonium ion analysis of fermented TVP

Ammonium ions of TVP fungal cultures were analyzed using ion chromatography (Compact IC, Metrohm). The conditions of the analysis are shown in Table 2 below.

**[Table 2]**

| IC system | Compact IC (Metrohm) |
|---|---|
| Column | Metriseo C4 column 150 mm × 4 mm (Cation) |
| Temperature | 25°C |
| Flow | 0.7 ml/min (Anion), 0.9 mL/min (Cation) |
| Detector | Conductivity |
| Solvent | A: 3.2 mM sodium carbonate |
| | B : 1 mM sodium bicarbonate (Anion) |
| | A: 0.7 mM dipicolinic acid, |
| | B: 1.7 mM nitric acid (Cation) |

### [Example 4] Results of texture and ammonium ion analysis of fermented TVP

In a TVP fungal culture in which *Rhizopus oligosporus* fungus was inoculated onto TVP, the mycelia bind to individual TVP grains to form a lump. In this example, fungi *Rhizopus oligosporus* was inoculated onto a mixed substrate produced by adding starch to TVP, and producing a fermented TVP by inoculating and culturing the fungi, and then, the flavor and texture of the fermented TVP were measured.

FIG. 1a shows images of the appearance before fermentation of a substrate in which TVP and starch were mixed in weight ratios of TVP:starch = 57:43 and 40:60, and FIG. 1b shows images of the appearance of *Rhizopus oligosporus* after its inoculation onto the substrate, culture, and fermentation. When starch was added in an excessive amount in a ratio of 60, exceeding 43 relative to that of TVP, it was confirmed that the shape of TVP grains or granules before fermentation was not well maintained (FIG. 1a), and this resulted in the nonuniform formation of fungal mycelia in the fermented TVP (FIG. 1b) .

The results of measuring the texture of the TVP fungal culture obtained by inoculating and culturing *Rhizopus oligosporus* into a mixture of TVP and carbohydrates, containing starch or sugar as a carbohydrate, are shown in Table 3. As shown in the results in Table 3, when the fungi was cultured by adding starch to TVP, the hardness and chewiness of the TVP fungal culture increased, thus confirming that the resulting TVP had a texture similar to commercially available beef patties. In addition, the content of ammonia (ammonium ions), which becomes a problem due to its negative flavor caused by the fermentation of the fungi, was further reduced when starch was mixed and fermented, thus confirming the potential of the resulting TVP as a food. This effect of starch on reducing the ammonia content was confirmed in all of the corn starch, potato starch, tapioca starch, and modified starch, such as pregelatinized corn starch and waxy corn gelatinized starch used in the experiment.

In contrast, the TVP fungal culture obtained by mixing sugar, which is a disaccharide, into TVP, inoculating it with *Rhizopus oligosporus,* followed by fermenting showed a lower ammonia content than the control group, in which starch was not added; however, its texture regarding hardness and chewiness were significantly lower, and thus, it was evaluated that its potential for use as a meat alternative such as a vegetable patty is not high. When glucose, which is a monosaccharide, was added, resulted in the quality deterioration due to browning during heat treatment for sterilization, and the growth of the *Rhizopus oligosporus* strain was reduced, and failure in forming a lump, thus making it difficult to use as a food.

**[Table 3]**

| Carbohyd rate Raw Material | Weight ratio of TVP:Carb ohydrate | Hardnes s (gf) | Elastic ity | Cohesiv eness | Chewi ness | Ammonium Ions (µg/g) |
|---|---|---|---|---|---|---|
| Control group | 100 : 0 | 369 | 0.59 | 0.46 | 103 | 5353 |
| Corn Starch | 87 : 13 | 483 | 0.60 | 0.40 | 121 | 2799 |
| | 73 : 26 | 1307 | 0.61 | 0.40 | 318 | 1631 |
| Potato Starch | 87 : 13 | 718 | 0.61 | 0.42 | 185 | 2933 |
| | 73 : 26 | 988 | 0.59 | 0.40 | 231 | 1748 |
| Tapioca Starch | 87 : 13 | 783 | 0.64 | 0.44 | 223 | 2241 |
| | 73 : 26 | 1325 | 0.72 | 0.44 | 410 | 959 |
| Pregelat inized Corn Starch | 87 : 13 | 602 | 0.62 | 0.35 | 132 | 2247 |
| | 73 : 26 | 984 | 0.56 | 0.36 | 202 | 1699 |
| Waxy Corn Pregelat inized Starch | 87 : 13 | 997 | 0.61 | 0.41 | 249 | 1487 |
| | 73 : 26 | 1231 | 0.60 | 0.33 | 245 | 988 |
| Glucose | 87 : 13 | 662 | 0.66 | 0.47 | 206 | 366 |
| | 73 : 26 | Not Measurable (No binding formation) | | | | |
| White Sugar | 87 : 13 | 284 | 0.52 | 0.38 | 57 | 3374 |
| | 73 : 26 | 247 | 0.49 | 0.38 | 48 | 2650 |

The results of the texture measurement of commercially available patties are shown in Table 4. The texture measurement was performed using the same method described in Example 2.

**[Table 4]**

| Commercial Products | Hardness (gf) | Elasticity | Cohesiveness | Chewiness |
|---|---|---|---|---|
| Beef Patty | 1441.9 | 0.62 | 0.40 | 359.6 |
| Wagyu Steak | 1823.69 | 0.68 | 0.42 | 513.90 |
| Charcoal BBQ Burger | 1067.10 | 0.60 | 0.42 | 267.82 |

In the above, representative Examples of the present application have been described by way of example, but the scope of the present application is not limited to the specific Examples described above. Anyone skilled in the art will be able to make appropriate changes within the scope of the claims of the present application.

## Claims

1. A method of producing a fermented texturized vegetable protein, comprising inoculating and culturing fungi on a substrate comprising a texturized vegetable protein (TVP) and starch.

2. The method of producing a fermented texturized vegetable protein according to claim 1, wherein the texturized vegetable protein (TVP) comprises proteins derived from wheat, soybeans, peas, sesame seeds, cotton seeds, or rice.

3. The method of producing a fermented texturized vegetable protein according to claim 1, wherein the starch is normal starch, pregelatinized starch, or modified starch.

4. The method of producing a fermented texturized vegetable protein according to claim 3, wherein the normal starch is starch extracted from one selected from the group consisting of wheat, rice, glutinous rice, mung beans, barley, soybeans, corn, sorghum, potato, sweet potato, tapioca, cassava, and arrowroot.

5. The method of producing a fermented texturized vegetable protein according to claim 1, wherein a weight ratio of the texturized vegetable protein (TVP) and starch is TVP: starch = 55:45 to 90:10.

6. The method of producing a fermented texturized vegetable protein according to claim 1, wherein the fungi are one or more fungi selected from the group consisting of *Rhizopus* genus, *Mucor* genus, *Neurospora* genus, *Amylomyces* genus, *Aspergillus* genus, and *Monascus* genus.

7. The method of producing a fermented texturized vegetable protein according to claim 6, wherein the fungi of *Rhizopus* genus is fungi of *Rhizopus oligosporus.*

8. The method of producing a fermented texturized vegetable protein according to claim 1, further comprising adding water to the substrate comprising the texturized vegetable protein (TVP) and starch to adjust a moisture content before inoculating the substrate with fungi.

9. The method of producing a fermented texturized vegetable protein according to claim 8, further comprising sterilizing the substrate after adjusting the moisture content by adding water to the substrate.

10. The method of producing a fermented texturized vegetable protein according to claim 9, wherein the sterilization is performed by heating at a temperature of 110°C to 125°C for 5 to 15 minutes.

11. A fermented texturized vegetable protein obtained by inoculating and culturing fungi on a substrate comprising a texturized vegetable protein (TVP) and starch.

12. The fermented texturized vegetable protein according to claim 11, wherein the fermented texturized vegetable protein comprises ammonium ions in a content of 5000 µg/g or less.

13. The fermented texturized vegetable protein according to claim 11, wherein the fermented texturized vegetable protein has one or more of the following textural characteristics:
(i) Hardness of 400 to 3000(gf),
(ii) Elasticity of 0.4 to 0.8,
(iii) Cohesiveness of 0.2 to 0.44, and
(iv) Chewiness of 121 to 900.

14. The fermented texturized vegetable protein according to claim 11, wherein the fermented texturized vegetable protein is produced by any one of the methods of claims 1 to 10.

15. A meat alternative comprising the fermented texturized vegetable protein according to any one of claims 11 to 13.

16. A method for reducing the off-flavor or off-odor of a fermented texturized vegetable protein, comprising inoculating and culturing fungi on a substrate comprising a texturized vegetable protein (TVP) and starch.

17. The method for reducing the off-flavor or off-odor of a fermented texturized vegetable protein according to claim 16, wherein the texturized vegetable protein (TVP) comprises proteins derived from wheat, soybeans, peas, sesame seeds, cotton seeds, or rice.

18. The method for reducing the off-flavor or off-odor of a fermented texturized vegetable protein according to claim 16, wherein the starch is normal starch, pregelatinized starch, or modified starch.

19. The method for reducing the off-flavor and off-odor of a fermented texturized vegetable protein according to claim 16, wherein the normal starch is starch extracted from one selected from the group consisting of wheat, rice, glutinous rice, mung beans, barley, soybeans, corn, sorghum, potato, sweet potato, tapioca, cassava, and arrowroot.

20. The method for reducing the off-flavor or off-odor of a fermented texturized vegetable protein according to claim 16, wherein a weight ratio of the texturized vegetable protein (TVP) and starch is TVP: starch = 55:45 to 90:10.

21. A use of a substrate comprising a texturized vegetable protein (TVP) and starch for reducing the off-flavor or off-odor of a fermented texturized vegetable protein, wherein the fermented texturized vegetable protein is produced by inoculating and culturing fungi on the substrate.

22. The use of a substrate comprising a texturized vegetable protein (TVP) and starch for reducing the off-flavor or off-odor of a fermented texturized vegetable protein according to claim 21, wherein the texturized vegetable protein (TVP) comprises proteins derived from wheat, soybeans, peas, sesame seeds, cotton seeds, or rice.

23. The use of a substrate comprising a texturized vegetable protein (TVP) and starch for reducing the off-flavor or off-odor of a fermented texturized vegetable protein according to claim 21, wherein the starch is normal starch, pregelatinized starch, or modified starch.

24. The use of a substrate comprising a texturized vegetable protein (TVP) and starch for reducing the off-flavor or off-odor of a fermented texturized vegetable protein according to claim 21, wherein the normal starch is starch extracted from one selected from the group consisting of wheat, rice, glutinous rice, mung beans, barley, soybeans, corn, sorghum, potato, sweet potato, tapioca, cassava, and arrowroot.

25. The use of a substrate comprising a texturized vegetable protein (TVP) and starch for reducing the off-flavor or off-odor of a fermented texturized vegetable protein according to claim 21, wherein the weight ratio of the texturized vegetable protein (TVP) and starch is TVP: starch = 55:45 to 90:10.
